(19) Europäisches Patentamt
European Patent Office
Office européen des brevets

(11) Veröffentlichungsnummer: **0 339 419 A2**

(12) **EUROPÄISCHE PATENTANMELDUNG**

(21) Anmeldenummer: **89106821.5**

(51) Int. Cl.⁴: **C07C 149/30**

(22) Anmeldetag: **17.04.89**

(30) Priorität: **29.04.88 DE 3814534**

(43) Veröffentlichungstag der Anmeldung:
**02.11.89 Patentblatt 89/44**

(84) Benannte Vertragsstaaten:
**AT BE DE FR GB IT NL SE**

(71) Anmelder: **BAYER AG**
**D-5090 Leverkusen 1 Bayerwerk(DE)**

(72) Erfinder: **Heywang, Gerhard, Dr.**
**Nittumer Weg 4**
**D-5060 Bergisch-Gladbach 2(DE)**
Erfinder: **Jonas, Friedrich, Dr.**
**Krugenofen 15**
**D-5100 Aachen(DE)**

(54) **Mercaptopyrene, ihre Herstellung und ihre Verwendung zur Herstellung von Charge-Transfer-Komplexen.**

(57) Die Erfindung betrifft Mercaptopyrene der Formel

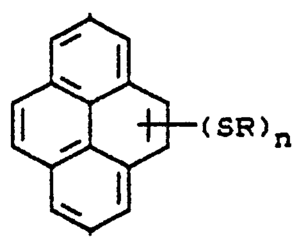

(I) ,

in der
n eine ganze Zahl von 3 bis 10 bedeutet und
R für einen gegebenenfalls substituierten Alkyl-, Alkenyl-, Alkinyl-, Cycloalkyl-, Aralkyl- oder Aryl-Rest steht, ein Verfahren zu ihrer Herstellung, ihre Verwendung zur Herstellung von CT-Komplexen und die erhaltenen CT-Komplexe.

EP 0 339 419 A2

Xerox Copy Centre

## Mercaptopyrene, ihre Herstellung und ihre Verwendung zur Herstellung von Charge-Transfer-Komplexen

Die Erfindung betrifft neue Mercaptopyrene, ihre Herstellung und ihre Verwendung zur Herstellung von elektrisch leitfähigen Salzen (Charge-Transfer-Komplexen) der Mercaptopyrene. (Die Charge-Transfer-Komplexe werden im folgenden abgekürzt als CT-Komplexe bezeichnet). Die Erfindung betrifft ferner die CT-Komplexe der Mercaptopyrene.

Es ist bereits bekannt, daß Pyren bei anodischer Oxidation in Gegenwart geeigneter Anionen elektrisch leitfähige Radikalkationensalze bildet (siehe Angew. Chem. 92, 941 (1980)) und beim Erhitzen mit Iod elektrisch leitfähige Pyren-Iod-Komplexe bildet (siehe J. Chem. Physics 34, 129 (1961)). Diese leitfähigen Pyren-Verbindungen haben jedoch den Nachteil, daß sie nicht stabil sind, sondern beim Aufbewahren an der Luft ihre Leitfähigkeit verlieren. Weiterhin ist bekannt, daß in Lösungen des Di-(methylmercapto)-pyrens bei aufeinanderfolgenden elektrochemischen Reduktionen und Oxidationen (zyklischer Voltammetrie) instabile Radikalkationen gebildet werden (siehe J. Org. Chem. 32, 1322 (1967)).

Es wurde nun gefunden, daß Mercaptopyrene, die drei und mehr Mercaptogruppen im Pyrenmolekül enthalten, auf einfache Weise zu Salzen (CT-Komplexen) oxidierbar sind und daß diese CT-Komplexe eine sehr gute elektrische Leitfähigkeit, eine hohe Stabilität und gute Verarbeitungseigenschaften aufweisen. Gegenüber den bislang bekannten CT-Komplexen des Tetracyanochinodimethans (TCNQ), des Tetrathiafulvalens und Bisethylenodithio-tetra-thiafulvalens stellen die aus den erfindungsgemäßen Mercaptopyrenen erhältlichen CT-Komplexe wegen ihrer leichteren Zugänglichkeit, höheren Stabilität und verbesserten Verarbeitbarkeit eine neue interessante Alternative dar.

Die Erfindung betrifft daher Mercaptopyrene der Formel

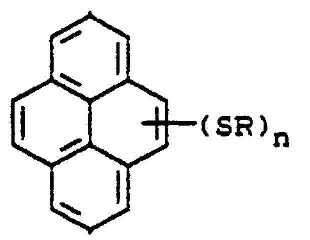

(I) ,

in der

n eine ganze Zahl von 3 bis 10, vorzugsweise 3 oder 4, bedeutet und

R für einen gegebenenfalls substituierten Alkyl-, Alkenyl-, Alkinyl-, Cycloalkyl-, Aralkyl- oder Aryl-Rest, vorzugsweise einen $C_1$-$C_{12}$-Alkyl-Rest steht.

Für R seien beispielsweise genannt:

als gegebenenfalls substituierte Alkylreste $C_1$-$C_{22}$-Alkyl-Reste wie der Methyl-, Ethyl-, n- und i-Propyl-, n-, sec.- und tert.-Butyl-, 2-Ethyl-hexyl-, n-Dodecyl-, Palmityl-, Stearyl- und Behenyl-Rest und durch Halogen, Hydroxy, $C_1$-$C_4$-Alkoxy, Nitro, Cyan, Carboxy, $C_1$-$C_4$-Alkoxycarbonyl, Acyl oder Amino substituierte $C_1$-$C_{12}$-Alkyl-Reste wie der 2-Chlor-, 2-Brom-, 2-Hydroxy-, 2-Methoxy-, 2-Cyanethyl-Rest, der Trifluorethyl-, Trichlormethyl-, Carboxymethyl-, Ethoxycarbonylmethyl-, Benzoylmethyl-Rest;

als gegebenenfalls substituierte Alkenylreste vor allem der Allyl- und Oleyl-Rest;

als gegebenenfalls substituierte Alkinylreste den Propargyl-Rest;

als gegebenenfalls substituierte Cycloalkyl-Reste vor allem der Cyclohexyl- und durch $C_1$-$C_4$-Alkyl oder Halogen substituierte Cyclohexyl-Reste wie der Methyl- und Dimethyl-cyclohexyl-, tert.-Butyl- und Chlorcyclohexyl-Rest;

als Aralkyl-Reste vorzugsweise der Benzyl- und 2-Phenylethyl-Rest und durch $C_1$-$C_4$-Alkyl, $C_1$-$C_4$-Alkoxy oder Halogen substituierte Benzyl-Reste wie der 4-Methylbenzyl-, 3-Chlorbenzyl- und der 4-Methoxybenzyl-Rest;

als gegebenenfalls substituierte Aryl-Reste vor allem der Phenyl und der durch $C_1$-$C_4$-Alkyl, $C_1$-$C_4$-Alkoxy oder Halogen substituierte Phenyl-Rest wie der Phenyl-, Tolyl-, Xylyl-, 5-Methoxyphenyl- und 2,4-Dichlorphenyl-Rest.

Bevorzugt sind für R gegebenenfalls durch $C_1$-$C_4$-Alkyl, $C_1$-$C_4$-Alkoxy und/oder Halogen substituierte $C_1$-$C_{22}$-Alkyl-, Benzyl- und Phenyl-Reste.

Als Vertreter der erfindungsgemäßen Mercaptopyrene der Formel I seien vor allem genannt:

1,3,6-Trimethylmercaptopyren, 1,3,6,8-Tetramethylmercaptopyren, 1,3,6,8-Tetraethylmercaptopyren, 1,3,6,8-Tetraisopropylmercaptopyren, 1,3,6,8-Tetrabutylmercaptopyren, 1,3,6,8-Tetrahexylmercaptopyren, 1,3,6,8-Tetraoctylmercaptopyren, 1,3,6,8-Tetradodecylmercaptopyren, 1,3,6,8-Tetrabenzylmercaptopyren, 1,3,6,8-Tetraphenylmercaptopyren.

Die erfindungsgemäßen Mercaptopyrene der Formel I werden durch Umsetzung von Halogenpyrenen der Formel

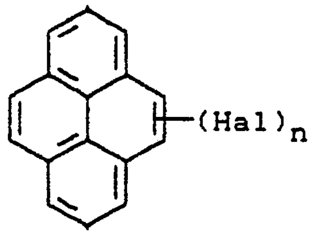

(II) ,

in der
n die unter Formel (I) angegebene Bedeutung hat und
Hal für Halogen, vorzugsweise für Chlor oder Brom steht,
mit Mercaptiden der Formel
$K^{m\oplus}$ $(SR)_m$ (III) ,
in der
R die unter Formel (I) angegebene Bedeutung hat und
$K^{m\oplus}$ für ein m-wertiges Kation, vorzugsweise ein Alkali-oder Erdalkali-Ion steht,
in einem stark polaren, aprotischen organischen Lösungsmittel bei Temperaturen von 0 bis 150° C, vorzugsweise 20 bis 150° C, erhalten.

Die Erfindung betrifft daher auch ein Verfahren zur Herstellung der Mercaptopyrene der Formel I, das dadurch gekennzeichnet ist, daß man Halogenpyrene der Formel II mit Mercaptiden der Formel III in einem stark polaren, aprotischen organischen Lösungsmittel bei Temperaturen von 0 bis 150° C, vorzugsweise 20 bis 150° C, umsetzt.

Die Halogenpyrene der Formel II und ihre Herstellung sind bekannt (siehe z.B. DE-OS 3 532 882 und Liebigs Ann. Chem. 531, 2 ff (1937).

Die Mercaptide der Formel III können als solche eingesetzt oder aber im Reaktionsgemisch in situ aus den entsprechenden Mercaptanen und Basen, z.B. Natriumhydrid oder Natriummethylat erzeugt werden.

Als stark polare, aprotische Lösungsmittel seien vor allem Dimethylformamid, Dimethylacetamid, N-Methylpyrrolidon, N-Methylcaprolactam, Tetramethylensulfon, Ethylencarbonat, Propylencarbonat, N,N′-Dimethylimidazolidinon und Tetramethylharnstoff genannt.

Die erfindungsgemäßen Mercaptopyrene der Formel I lassen sich chemisch oder elektrochemisch zu elektrisch leitfähigen Salzen (CT-Komplexen) oxidieren; die Zusammensetzung dieser CT-Komplexe läßt sich durch die Formel

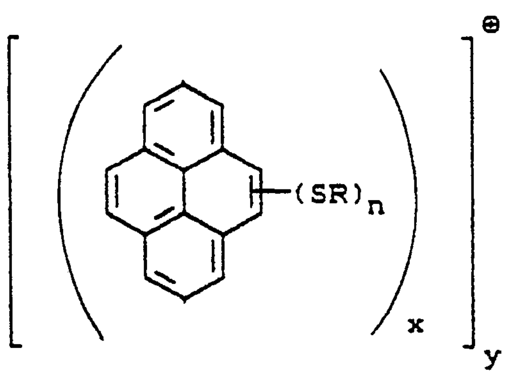

$A^{y\ominus}$ (IV) ,

beschreiben, in der
R und n die unter Formel I angegebene Bedeutung haben,
x die Gesamtzahl der in einem 1-wertigen Mercaptopyren-Kation enthaltenen neutralen und geladenen Mercaptopyren-Einheiten angibt und eine ganze oder gebrochene Zahl von 1 bis 10, vorzugsweise 1 bis 5,

ist,
$A^{\ominus}$ für ein Anion steht und
y die Zahl der negativen Ladungen (das heißt die negative Wertigkeit) des Anions und die zur Neutralisation dieser negativen Ladungen erforderliche Zahl 1-wertiger Mercaptopyren-Kationen bedeutet und eine ganze Zahl von 1 bis 3, vorzugsweise 1, ist.

Als Anionen kommen einwertige Anionen wie das $Cl^{\ominus}$, $Br^{\ominus}$, $J^{\ominus}$, $J_3{}^{\ominus}$, $HSO_4{}^{\ominus}$, das Methosulfat-, Tosylat-, Perchlorat-, Tetrafluoroborat- oder Hexafluorophosphation, aber auch zweiwertige Ionen wie das Sulfat- und dreiwertige Ionen wie das Phosphation in Betracht. Bevorzugt sind einwertige Anionen wie das Methosulfat-, Hexafluorophosphat-, Tetrafluoroborat-, Perchlorat-, $J_3{}^{\ominus}$-, $Br_3{}^{\ominus}$- und das Tosylat-Anion.

Die Oxidation der erfindungsgemäßen Mercaptopyrene kann sowohl elektrochemisch als auch chemisch erfolgen. Die elektrochemische Oxidation wird in inerten Lösungsmitteln in Gegenwart von Leitsalzen vorgenommen.

Als inerte Lösungsmittel werden vorzugsweise verwendet: Nitrile wie Acetonitril, Propionitril und Butyronitril; Amide wie Dimethylformamid und Dimethylacetamid, Harnstoffe wie Tetramethylharnstoff, Carbonate, wie 1,3-Dioxa-cyclohexanon-(2) und 1,3-Dioxa-4-methyl-cyclopentanon-(2); Lactone und Lactame wie Butyrolacton und N-Methyl-pyrrolidon und Caprolactam; Sulfoxide und Sulfone wie Dimethylsulfoxid und Dimethylsulfon und Tetramethylensulfon; aromatische Kohlenwasserstoffe wie Toluol, Xylol und Chlorbenzol, Ketone wie Aceton oder Cyclohexanon; Alkohole wie Methanol und Ethanol; halogenierte Kohlenwasserstoffe wie Methylenchlorid und 1,2-Dichlorethan. Auch Wasser und anorganische Lösungsmittel wie flüssiges Schwefeldioxid können verwendet werden.

Als Leitsalze werden die Fluoride, Chloride, Bromide, Iodide, Monoalkylsulfate, Perchlorate, Tetrafluoroborate, Hexafluorophosphate, Hexafluorantimonate, Hexafluoroarsenate, Methansulfonate, Trifluormethansulfonate, Benzolsulfonate, Tosylate, Benzoate und Acetate von Alkali- und Erdalkali-Metallen oder von Kupfer und Silber, ferner von Oniumverbindungen verwendet, die durch erschöpfende Alkylierung von Arsinen, Phosphinen, Thioethern oder tertiären Aminen erhalten wurden. Diese Oniumsalze können unmittelbar in der Form verwendet werden, in der sie bei der erschöpfenden Alkylierung anfallen. Man kann in ihnen aber auch zunächst das Anion gegen ein anderes Anion austauschen, z.B. im Tetrabutylammoniumchlorid, das Chloridion gegen das Tetrafluoroboration.

Die elektrochemische Oxidation wird bei Temperaturen von -78° C bis zum Siedepunkt des verwendeten Lösungsmittels vorgenommen. Bevorzugt sind Temperaturen von 0 bis 100° C, insbesondere von 20 bis 85° C.

Die Konzentrationen der Mercaptopyrene in den zu elektrolysierenden Lösungen liegen zwischen 0,001 Mol/l und der Sättigungskonzentration des betreffenden Mercaptopyrens im jeweiligen Lösungsmittel bei der angewandten Temperatur. Bevorzugt sind Konzentrationen von 0,005 bis 0,02 Mol Mercaptopyren je Liter Lösung.

Die Leitsalze können im zwei- bis zwanzigfachen molaren Überschuß, bezogen auf eingesetztes Mercaptopyren verwendet werden. Bevorzugt werden 3 bis 12 Mol Leitsalz je Mol Mercaptopyren eingesetzt.

Die elektrochemische Oxidation kann potentiostatisch oder galvanostatisch durchgeführt werden. Bevorzugt ist die galvanostatische Arbeitsweise. In einem typischen galvanostatischen Experiment wird in einer 100 ml-Zelle mit zwei in einem Abstand von 1 cm angeordneten 16 $cm^2$-Pt-Elektroden bei einer Stromstärke von 1,5 mA und einer Zellspannung zwischen 0,7 und 5 V elektrolysiert. Gegebenenfalls können Anoden- und Kathodenraum auch durch eine Membrane oder Fritte getrennt sein.

Die erfindungsgemäßen CT-Komplexe der Mercaptopyrene schedien sich während der Elektrolyse an der Anode ab und werden in an sich bekannter Weise durch mechanisches Abtrennen, Waschen mit einem der vorstehend erwähnten inerten Lösungsmittel und Trocknen analysenrein gewonnen.

Bei der chemischen Oxidation werden die erfindungsgemäßen Mercaptopyrene mit üblichen Oxidationsmitteln umgesetzt. Die Menge an Oxidationsmittel wird dabei so bemessen, daß auf 1 Mol Mercaptopyren 0,1 bis 5 Äquivalente Oxidationsmittel entfallen. Durch die Wahl der Menge an Oxidationsmittel läßt sich die Zahl "x", die die Gesamtzahl der im 1-wertigen Mercaptopyren-Kation enthaltenen Mercaptopyren-Einheiten beeinflussen. Je geringer die Menge an Oxidationsmittel umso größer die Zahl der neutralen Mercaptopyren-Einheiten im Mercaptopyren-Kation.

Die Oxidation der erfindungsgemäßen Mercaptopyrene wird in Gegenwart flüssiger Verdünnungsmittel vorgenommen. Die Mercaptopyrene können in diesem flüssigen Medium vollständig oder teilweise gelöst oder aber auch nur suspendiert vorliegen.

Für die Oxidation der erfindungsgemäßen Mercaptopyrene der Formel I eignen sich sowohl die für chemische Oxidationen chemischer Verbindungen üblicherweise verwendeten Oxidationsmittel wie $H_2O_2$, Caro'sche Säure, Perborate, Peroxydisulfate, Perbenzoesäure, Ozon, Halogene wie Chlor, Brom oder Iod,

Kaliumpermanganat, Kaliumchromat, Kaliumdichromat; für die oxidative Polymerisation hete rocyclischer Verbindungen zu elektrisch leitfähigen Polymeren verwendete Oxidationsmittel wie Eisen-(III)-salze wie Fe-(III)-chlorid, Fe-(III)-tosylat, Fe-(III)-perchlorat, Fe-(III)-4-dodecylbenzolsulfonat, ferner Nitroniumhexafluorophosphat, -arsenat, -antimonat, Nitroniumtetrafluoroborat, Nitrosoniumhexafluorophosphat, -arsenat, -antimonat, Nitrosoniumtetrafluoroborat; und für die oxidative Dotierung (p-Dotierung) von Polyacetylenen und Polysulfiden zu elektrisch leitfähigen Polyacetylenen und Polysulfiden verwendete Oxidationsmittel wie Arsenpentafluorid, Antimonpentafluorid, Arsenpentachlorid, Antimonpentachlorid, Aluminiumchlorid, Zinntetrachlorid und Zinntetraiodid.

Als Verdünnungsmittel werden vorzugsweise organische Lösungsmittel verwendet, die ein möglichst gutes Lösungsvermögen für die Mercaptopyrene aufweisen.

Durch die Anwesenheit weiterer Salze oder Säuren in der Reaktionslösung können deren durch Dissoziation gebildeten Anionen in die CT-Komplexe der Mercapto-substituierten Pyrene eingebaut werden.

Die chemische Oxidation wird bei Temperaturen von 0 bis 200° C, vorzugsweise im Bereich von 0° C bis zum Siedepunkt des verwendeten Lösungsmittels vorgenommen, gegebenenfalls kann auch unter Druck gearbeitet werden.

Die durch die Oxidation der erfindungsgemäßen Mercaptopyrene erhältlichen CT-Komplexe sind wertvolle organische elektrisch leitfähige Verbindungen. Sie eignen sich zur Antistatik-Ausrüstung von Kunststoffen und als organische Leiter auf dem Elektroniksektor zur Übertragung von elektrischen Signalen und elektrischer Energie.

Die in den folgenden Beispielen für die CT-Komplexe angegebenen elektrischen Leitfähigkeiten [S/cm] wurden, sofern nichts anderes angegeben ist, an Pulverpreßlingen nach der Vierelektrodenmethode bestimmt.

Beispiel 1

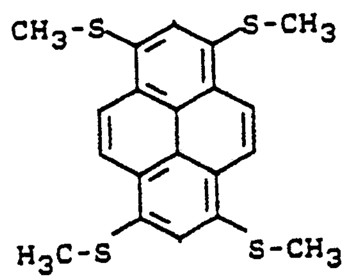

a) In die in einem mit Rührer, Gaseinleitungsrohr und Rückflußkühler versehenen 2000 ml Dreihalskolben befindliche Suspension von 12 g (0,4 Mol) Natriumhydrid (80 %ig in Paraffin) in 200 ml 1,3-Dimethylimidazolidinon-2 werden bei 0° C langsam unter Rühren 19,2 g (0,4 Mol) Methylmercaptan eingeleitet. In die Suspension von Natriummethylmercaptid werden anschließend unter Rühren 25 g (0,05 Mol) 1,3,6,8-Tetrabrom-pyren eingetragen. Das Reaktionsgemisch wird 1 Stunde bei Raumtemperatur, dann 24 Stunden bei 40° C gerührt. Anschließend wird der gelbe Niederschlag abgesaugt, mit Wasser und Methanol gewaschen und 1 Stunde bei 100° C im Vakuum getrocknet.

Es werden 18,9 g (98 % der Theorie) 1,3,6,8-Tetramethylmercaptopyren in Form eines gelben Pulvers erhalten. FP.: 275-280° C (Sintern ab 250° C).

b) In die in einem mit Rührer, Gaseinleitungsrohr und Rückflußkühler versehenen 500 ml-Dreihalskolben befindliche Lösung von 10,8 g (0,2 Mol) Natriummethylat in 100 ml Dimethylformamid werden bei 0° C langsam unter Rühren 9,6 g (0,2 Mol) Methylmercaptan eingeleitet. Die auf diese Weise erhaltene Suspension von Natriummethylmercaptid in Dimethylformamid wird unter Rühren mit 12,5 g (0,025 Mol) 1,3,6,8-Tetrabrompyren versetzt. Das Reaktionsgemisch wird 1 Stunde bei Raumtemperatur und 24 Stunden bei 40° C gerührt. Anschließend wird der gelbe Niederschlag abgesaugt, mit Wasser und Methanol gewaschen und 1 Stunde bei 100° C im Vakuum getrocknet.

Es werden 9,6 g (= 99,5 % der Theorie) 1,3,6,8-Tetramethylmercaptopyren in Form eines gelben Pulvers erhalten. FP.: 275-280° C, (Sintern ab 250° C).

c) Durch Einleiten von Methylmercaptan in die Lösung von Natriummethylat in Methanol wird nach dem Entfernen des Lösungsmittels und Trocknen des Rückstandes Natriummethylmercaptid in Form eines farblosen, feinen, rieselfähigen Pulvers erhalten.

29 g (0,41 Mol) dieses Natriummethylmercaptids werden in 800 ml Dimethylformamid gelöst. Die Lösung wird mit 46,6 g (0,09 Mol) 1,3,6,8-Tetrabrompyren versetzt. Die Lösung färbt sich rasch intensiv gelb. Das Reaktionsgemisch wird 15 Stunden bei 100° C gerührt und anschließend mit 800 ml Wasser versetzt. Der Niederschlag wird abfiltriert, mit Methanol und Wasser gewaschen und im Hochvakuum getrocknet.

Es werden 31,5 g (= 91 % der Theorie) 1,3,6,8-Tetramethylmercaptopyren in Form eines gelben Pulvers erhalten. FP.: 275-280° C (Sintern ab 250° C).

Beispiel 2

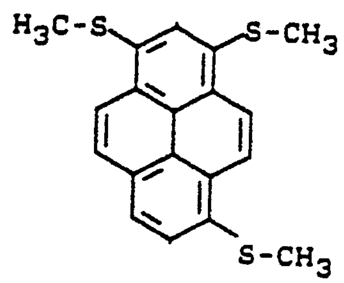

11 g (0.15 Mol) Natriummethylmercaptid werden mit 22 g (0,05 Mol) 1,3,6-Tribrompyren in 150 ml N,N'-Dimethylimidazolidinon-2 in der in Beispiel 1 c) beschriebenen Weise umgesetzt.

Es werden 15,1 g (= 88 % der Theorie) 1,3,6-Trimethylmercaptopyren in Form eines gelben Pulvers erhalten; FP.: 235-240° C.

Beispiel 3

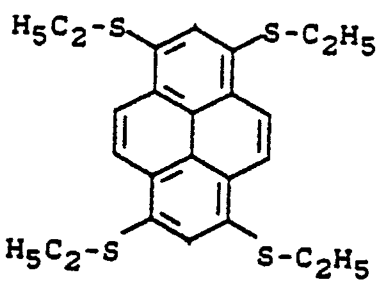

In dem in Beispiel 1 a) beschriebenen Reaktionsgefäß wird die Suspension von 12 g (0,4 Mol) Natriumhydrid (80 %ig in Paraffin) in 100 ml 1,3-Dimethylimidazolidinon-2 bei 0° C langsam mit 24,8 g (0,4 Mol) Ethylmercaptan versetzt. Anschließend wird das Reaktionsgemisch unter Rühren mit 25,9 g (0,05 Mol) 1,3,6,8-Tetrabrompyren versetzt. Das Reaktionsgemisch wird 1 Stunde bei Raumtemperatur und 24 Stunden bei 40° C gerührt. Anschließend wird der gelbe Niederschlag abgesaugt, mit Wasser und Methanol gewaschen und 1 Stunde bei 100° C im Vakuum getrocknet.

Es werden 20 g (= 90,5 % der Theorie) 1,3,6,8-Tetraethylmercaptopyren in Form eines orangeroten Pulvers erhalten; FP.: 180° C.

Beispiel 4

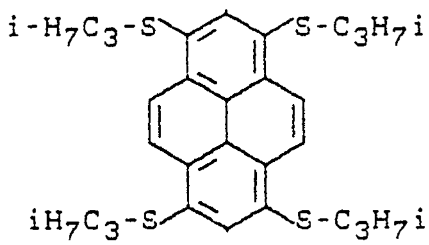

19,6 g (0,2 Mol) Natriumisopropylmercaptid und 26 g (0,05 Mol) 1,3,6,8-Tetrabrompyren werden in 200 ml N,N′-dimethylimidazolidinon-2 5 Tage bei Raumtemperatur gerührt. Anschließend wird das Reaktionsgemisch in Wasser eingetragen und der Niederschlag abgesaugt und aus Ethanol umkristallisiert.

Es wurden 7 g (= 28 % der Theorie) 1,3,6,8-Tetraisopropylmercaptopyren in Form eines gelben Kristallpulvers erhalten; FP.: 108° C.

Beispiel 5

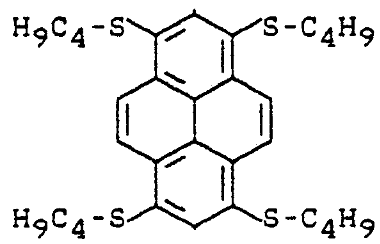

22,4 g (0,2 Mol) Natriumbutylmercaptid und 20,7 g (0,04 Mol) 1,3,6,8-Tetrabrompyren werden in 250 ml N,N′-Dimethylimidazolidinon-2 1 Stunde bei 80° C gerührt. Der Niederschlag wird abgesaugt und mit Wasser und Methanol gewaschen und im Hochvakuum getrocknet.

Es werden 17,5 g (= 79 % der Theorie) 1,3,6,8-Tetrabutyl-mercaptopyren in Form eines gelben, fluoreszierenden Kristallpulvers erhalten; FP.: 91-92° C.

Beispiel 6

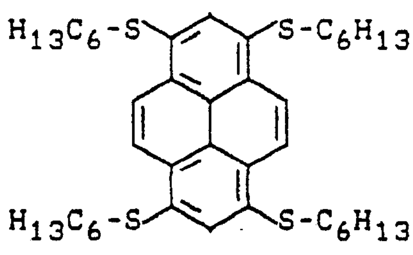

8,4 g (0,06 Mol) Natriumhexylmercaptid und 5,2 g (0,01 Mol) 1,3,6,8-Tetrabrompyren werden in 100 ml N,N′-Dimethylimidazolidinon-2 2 Stunden bei 80° C gerührt. Der Niederschlag wird abgesaugt und mit Wasser und Methanol gewaschen und im Hochvakuum getrocknet.

Es werden 2,5 g (= 37 % der Theorie) 1,3,6,8-Tetrahexyl-mercaptopyren in Form eines gelben, fluoreszierenden Kristallpulvers erhalten; FP.: 92° C.

Beispiel 7

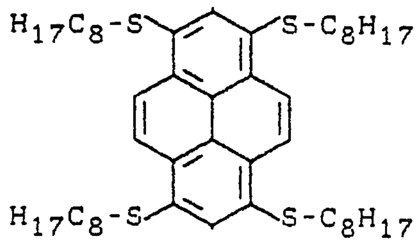

10,2 g (0,06 Mol) Natriumoctylmercaptid und 5,2 g (0,01 Mol) 1,3,6,8-Tetrabrompyren werden in 100 ml N,N′-Dimethylimidazolidinon-2 1 Stunde bei 80° C gerührt. Der Niederschlag wird abgesaugt und mit Wasser und Methanol gewaschen und im Hochvakuum getrocknet.

Es werden 4,2 g (= 53 % der Theorie) 1,3,6,8-Tetraoctyl-mercaptopyren in Form eines gelben, fluoreszierenden Kristallpulvers erhalten; FP.: 110° C.

Beispiel 8

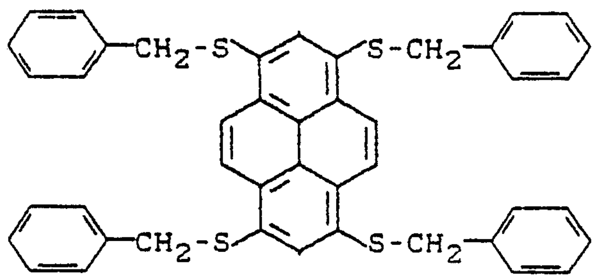

29,4 g (0,2 Mol) Natriumbenzylmercaptid und 20,7 g (0,04 Mol) 1,3,6,8-Tetrabrompyren werden in 250 ml N,N′-Dimethylimidazolidinon-2 2 Stunden bei 80° C gerührt. Der Niederschlag wird abgesaugt und mit Wasser und Methanol gewaschen und im Hochvakuum getrocknet.

Es werden 23 g (= 83 % der Theorie) 1,3,6,8-Tetrabenzyl-mercaptopyren in Form eines gelben, fluoreszierenden Kristallpulvers erhalten; FP.: 178-180° C.

Beispiel 9

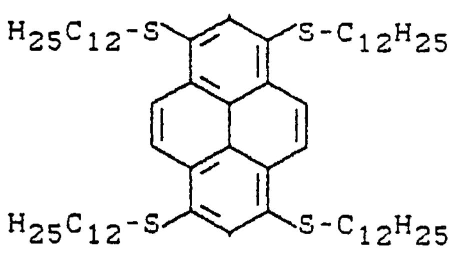

26,9 g (0,12 Mol) Natriumdodecylmercaptid und 13 g (0,025 Mol) 1,3,6,8-Tetrabrompyren werden in 500 ml N,N′-Dimethylimidazolidinon-2 14 Stunden bei 80° C gerührt. Der Niederschlag wird abgesaugt und mit Wasser und Methanol gewaschen und im Hochvakuum getrocknet.

Es werden 15,5 g (= 60 % der Theorie) 1,3,6,8-Tetradodecylmercaptopyren in Form eines gelben, fluoreszierenden Kristallpulvers erhalten; FP.: 110° C.

Beispiel 10

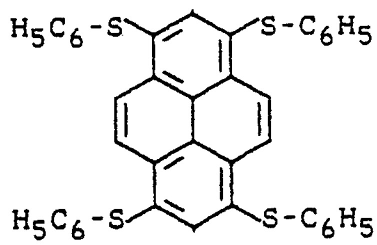

Zu der in einem mit Rührer, Tropftrichter und Rückflußkühler versehenen 500 ml Dreihalskolben befindlichen Lösung von 9,2 g (0,4 Mol) Natrium in 200 ml Ethanol werden unter Rühren 44 g (0,4 Mol) Thiophenol getropft. Die Natriumthiophenolatlösung wird im Vakuum zur Trockne eingeengt und der Rückstand in 100 ml 1,3-Dimethylimidazolidinon-2 suspendiert. Die Suspension wird mit 25,9 g (0,05 Mol) 1,3,6,8-Tetrabrompyren versetzt, 1 Stunde bei Raumtemperatur und 24 Stunden bei 40° C gerührt. Der Gelbe Niederschlag wird abgesaugt, mit Wasser und Methanol gewaschen und 1 Stunde bei 100° C im Vakuum getrocknet.

Es werden 31,5 g (= 99,4 % der Theorie) 1,3,6,8-Tetraphenylmercaptopyren erhalten; FP.: 230° C.

Beispiele 11 bis 16

(Herstellung von CT-Komplexen der erfindungsgemäßen Mercaptopyrene durch anodische Oxidation)

Allgemeine Arbeitsweise:

In einer mit zwei 16 $cm^2$-Pt-Elektroden ausgerüsteten 100 ml fassenden Elektrolysezelle (Abstand der Elektroden voneinander: 1 cm) wurde eine Lösung von 1 mMol Tetramethylmercaptopyren und 5 mMol Leitsalz in 100 ml Nitrobenzol bei einer Stromstärke von 1,5 mA elektrolysiert. In der nachstehenden Tabelle sind die erhaltenen Mercaptopyren-CT-Komplexe, die für die Oxidation verwendeten Leitsalze, die angewandte Elektrolysedauer und -temperatur und die elektrischen Leitfähigkeiten der CT-Komplexe (Pulver) und die Ausbeuten, in denen sie erhalten wurden, zusammengestellt.

Tabelle

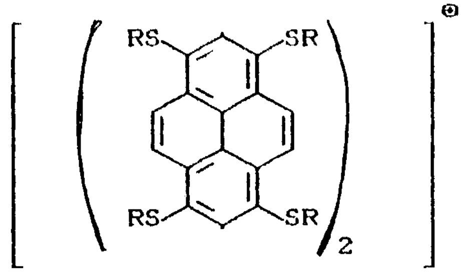

$A^{\ominus}$

| Beispiel | R | Leitsalz | $A^{\ominus}$ | Elektrolyse Temperatur [°C] | sich einstellende Spannung [V] | Elektrolyse Dauer [h] | elektr. Leitfähigkeit des CT-Komplexes [S/cm] | Ausbeute [% der Theorie] |
|---|---|---|---|---|---|---|---|---|
| 11 | $CH_3$ | 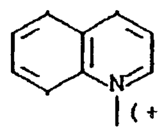 (+) $CH_3$-$^{(-)}OSO_2$-$OCH_3$ | $CH_3OSO_3^-$ | 80 | 1,4 | 24 | $3,1 \times 10^{-3}$ | 58 |
| 12 | $CH_3$ | $[(C_4H_9)_4N]^+ClO_4^-$ | $ClO_4^-$ | 80 | 1,6 | 26 | $4,7 \times 10^{-2}$* | 22,3 |
| 13 | $CH_3$ | $[(C_4H_9)_4N]^+PF_6^-$ | $PF_6^-$ | 80 | 1,5 | 26 | $3,7 \times 10^{-2}$ | 34 |
| 14 | $CH_3$ | $[(C_4H_9)_4N]^+BF_4^-$ | $BF_4^-$ | 80 | 1,6 | 24 | $2,4 \times 10^{-2}$ | 20,3 |
| 15 | $C_2H_5$ | $[(C_4H_9)_4N]^+PF_6^-$ | $PF_6^-$ | 20 | 3,7 | 24 | $3,5 \times 10^{-3}$ | 27 |
| 16 | $C_2H_5$ | $[(C_4H_9)_4N]^+BF_4^-$ | $BF_4^-$ | 20 | 2,4 | 24 | $4,6 \times 10^{-3}$ | 21,7 |

* Die Kristall-Leitfähigkeit des CT-Komplexes beträgt in Abhängigkeit von der Kristallqualität 250 bis 600 S/cm

Beispiel 17

a) 386 mg (1 mMol) 1,3,6,8-Tetramethylmercaptopyren und 635 mg (5 mMol) Iod werden in 60 ml 1,2,4-Trichlorbenzol auf Rückflußtemperatur erhitzt. Nach dem Abkühlen auf Raumtemperatur wird der Niederschlag abgesaugt.

Es werden 380 mg (= 74,1 % der Theorie) des CT-Komplexes der Formel

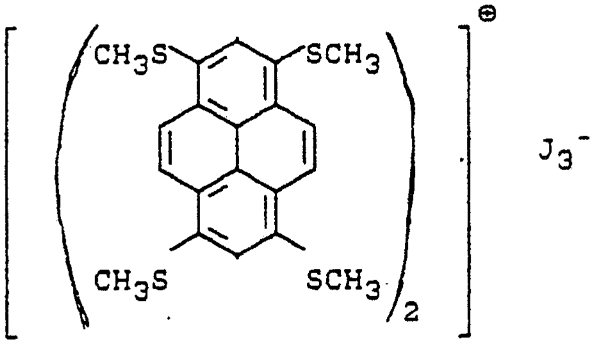

in Form feiner, verfilzter, schwarzgoldener Kristallnädelchen erhalten. Die elektrische Leitfähigkeit des CT-Komplexes beträgt 8,7 S/cm, seine Zersetzungstemperatur 240° C.

b) Bei gleicher Arbeitsweise, aber Verwendung von 1,3,6,8-Tetradodecylmercaptopyren, wird der CT-Komplex der Formel

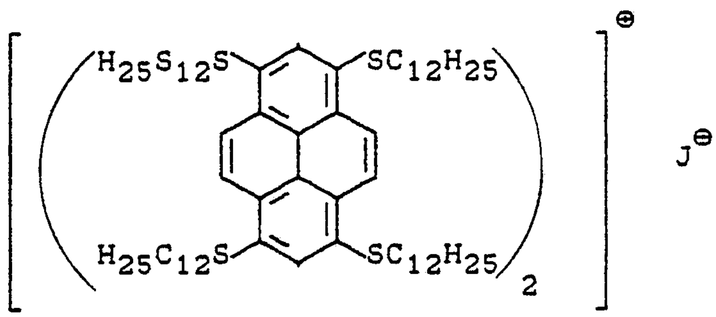

in Form eines olivgrünen Kristallpulvers erhalten. Die elektrische Leitfähigkeit dieses CT-Komplexes beträgt $3 \times 10^{-3}$ S/cm; FP.: 106-108° C.

Die Leitfähigkeit des Komplexes wird durch das Schmelzen nicht beeinträchtigt.

Beispiel 18

a) Die heiße Lösung von 772 mg (2 mMol) 1,3,6,8-Tetramethylmercaptopyren in 10 ml 1,2,4-Trichlorbenzol wird mit der heißen Lösung von 254 mg (2 mMol) Iod in 10 ml 1,2,4-Trichlorbenzol versetzt. Aus dem Reaktionsgemisch fallen sofort schwarze Kristalle aus. Nach dem Abkühlen auf Raumtemperatur wird der Niederschlag abgesaugt und erst mit wenig 1,2,4-Trichlorbenzol, dann mit Methylenchlorid gewaschen.

Es werden 720 mg (= 70,2 % der Theorie) des CT-Komplexes der in Beispiel 13a angegebenen Formel in Form schwarzer Kristalle mit goldmetallischem Glanz erhalten. Die elektrische Leitfähigkeit der Kristalle beträgt 3,2 S/cm.

b) Bei gleicher Arbeitsweise, aber Verwendung von 1,3,6,8-Tetraethylmercaptopyren, wird ein entsprechender CT-Komplex mit Ethylmercapto-Gruppen erhalten. Die Leitfähigkeit dieses Komplexes beträgt 0,7 S/cm.

Beispiel 19

a) In die Lösung von 164 mg Iod (1,3 mMol) in 10 ml Methylenchlorid werden bei 20° C 500 mg (1,3 mMol) 1,3,6,8-Tetramethylmercaptopyren eingetragen. Das Reaktionsgemisch färbt sich sofort schwarz. Nach 2-stündigem Stehen bei Raumtemperatur wird der Niederschlag abgesaugt und mit Methylenchlorid gewaschen.

Es werden 523 mg (= 78,4 % der Theorie) des CT-Komplexes der Formel

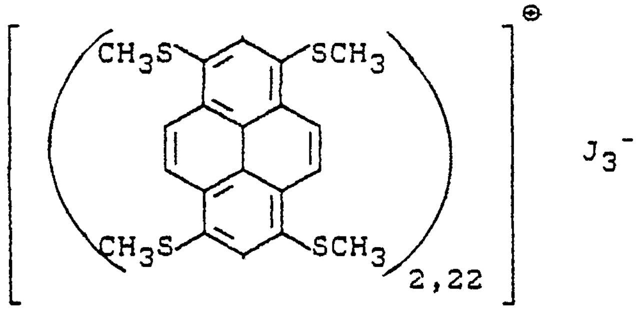

in Form dunkler Kristalle mit goldmetallischem Glanz erhalten. Die elektrische Leitfähigkeit des CT-Komplexes beträgt 0,3 S/cm.

b) Bei gleicher Arbeitsweise, aber Verwendung von 1,3,6,8-Tetrabutylmercaptopyren, wird der CT-Komplex der Formel

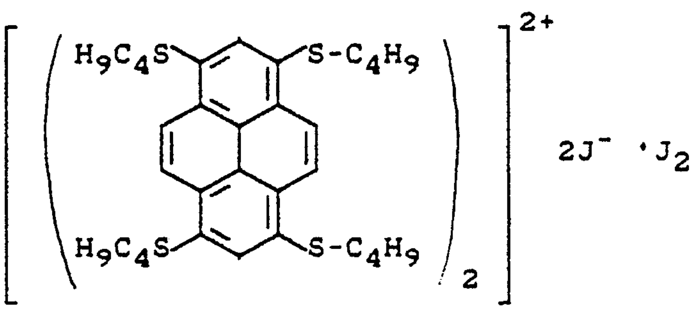

in Form eines schwarzen Kristallpulvers erhalten.

Die elektrische Leitfähigkeit dieses CT-Komplexes beträgt 0,16 S/cm. Der Komplex schmilzt bei 120° C unter Abgabe von Joddämpfen.

Beispiel 20

1 g (1 mMol) 1,3,6,8-Tetradodecylmercaptopyren und 380 mg (1,5 mMol) Iod werden bei 100° C miteinander verschmolzen. Nach dem Abkühlen wird das überschüssige Iod aus der Schmelze mit Methylenchlorid extrahiert. Die Leitfähigkeit des auf diese Weise erhaltenen CT-Komplexes der in Beispiel 17b angegebenen Formel beträgt 5 x $10^{-3}$ S/cm. Selbst durch viermaliges Aufschmelzen des Komplexes wird seine Leitfähigkeit nicht herabgesetzt.

Beispiel 21

a) Die Suspension von 1,93 g (5 mMol) 1,3,6,8-Tetramethylmercaptopyren in 40 ml Methylenchlorid wird mit der Lösung von 0,8 g (5 mMol) Brom in 10 ml Methylenchlorid versetzt. Der Niederschlag wird nach 2-stündigem Aufbewahren des Reaktionsgemisches bei Raumtemperatur abgesaugt und mit Methylenchlorid gewaschen.

Es werden 2,5 g (=63 % der Theorie) des CT-Komplexes der Formel

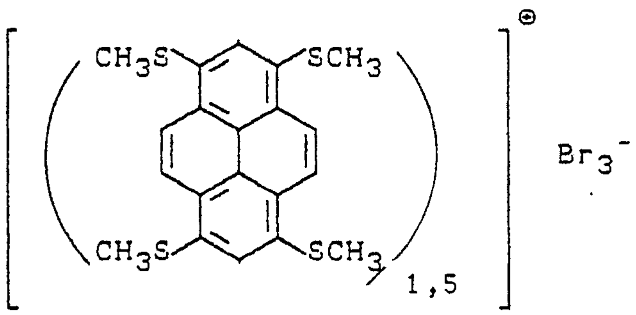

in Form schwarzer Kristalle erhalten. Die Leitfähigkeit dieses CT-Komplexes beträgt 3,36 S/cm.

b) Bei gleicher Arbeitsweise, aber Verwendung von 1,3,6-Trimethylmercaptopyren, wird ein entsprechender CT-Komplex mit einem durch 3 Methylmercapto-Gruppen substituierten Pyren erhalten. Die Leitfähigkeit dieses CT-Komplexes beträgt 1,2 S/cm.

c) Bei gleicher Arbeitsweise, aber Verwendung von 1,3,6,8-Tetradodecylmercaptopyren, wird ein entsprechender CT-Komplex mit einem durch 4 Dodecylmercapto-Gruppen substituierten Pyren erhalten. Die Leitfähigkeit dieses CT-Komplexes beträgt 3,1 x $10^{-3}$ S/cm.

Beispiel 22

Die Lösung von 152 mg (1 mMol) Eisen-(III)-chlorid (wasserfrei) in 10 ml Acetonitril wird bei 20° C mit 386 mg (1 mMol) 1,3,6,8-Tetramethylmercaptopyren versetzt. Das Reaktionsgemisch färbt sich schnell dunkel. Nach 18-stündigem Rühren bei Raumtemperatur wird der Niedrschlag abgesaugt und mit Acetonitril gewaschen.

Es werden 390 mg (= 71,2 % der Theorie) des Charge-Transfer-Komplexes der Formel

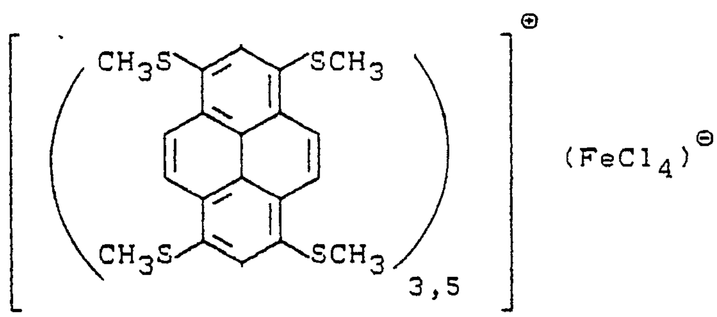

in Form schwarzer Kristalle erhalten. Die Leitfähigkeit des CT-Komplexes beträgt 0,35 S/cm.

Beispiel 23

a) Die Lösung von 386 mg (1 mMol) 1,3,6,8-Tetramethylmercaptopyren in 10 ml Acetonitril wird mit einer Lösung von 3 g (5,2 mMol) Eisen-(III)-tosylat in 10 ml Acetonitril versetzt. Das Reaktions gemisch färbt sich langsam dunkel. Nach 12-stündigem Stehen bei Raumtemperatur wird der Niederschlag abfiltriert, mit Acetonitril gewaschen und getrocknet.

Es werden 0,72 g (= 76 % der Theorie) des CT-Komplexes der Zusammensetzung

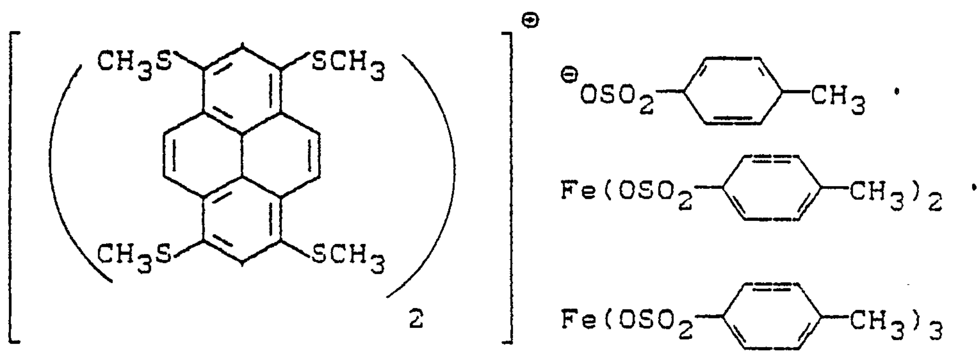

in Form olivgrüner Kristallnadeln erhalten. Die elektrische Leitfähigkeit dieser Kristalle beträgt 7,6 x $10^{-3}$ S/cm.

b) Bei gleicher Arbeitsweise, aber Verwendung von 1,87 g (2 mMol) Eisen-(III)-(4-dodecylbenzolsulfonat) als Oxidationsmittel, wird ein CT-Komplex erhalten, der eine Leitfähigkeit von 2,3 x $10^{-3}$ S/cm aufweist und der bei 230° C schmilzt. Die Leitfähigkeit dieses Komplexes wird durch die mit dem Schmelzen verbundene thermische Belastung praktisch nicht verändert.

c) Bei gleicher Arbeitsweise, aber Verwendung von 1 g (1 mMol) 1,3,6,8-Tetradodecylmercaptopyren und 0,7 g (1,2 mMol) Eisen-(III)-tosylat, wird ein gelbgrüner CT-Komplex erhalten, dessen Leitfähigkeit 1,4 x $10^{-6}$ S/cm beträgt.

d) Bei gleicher Arbeitsweise, aber Verwendung von 1 g (1 mMol) 1,3,6,8-Tetradodecylmercaptopyren und unter Verwendung von 1,8 g (1,7 mMol) Eisen-(III)-(4-dodecylbenzol)-sulfonat als Oxidationsmittel, wird ein olivgrüner CT-Komplex erhalten, der zwar nur eine Leitfähigkeit von 6 x $10^{-7}$ S/cm, aber dafür die bemerkenswerte Eigenschaft aufweist, durch Druck verformbar zu sein.

Beispiel 24

Die Suspension von 193 mg (0,5 mMol) 1,3,6,8-Tetramethylmercaptopyren und 171 mg (0,5 mMol) Tetrabutylammoniumperchlorat in 10 ml Tetrahydrofuran wird mit 10 Tropfen $H_2O_2$ (35 %ig) versetzt. Anschließend wird das Reaktionsgemisch mit 10 Tropfen konzentrierter Schwefelsäure angesäuert. Dabei färbt es sich schwarz. Der Niederschlag wird abgesaugt, mit Wasser und Methanol gewaschen und getrocknet.

Es werden 152 mg (= 70,2 % der Theorie) des CT-Komplexes der Formel

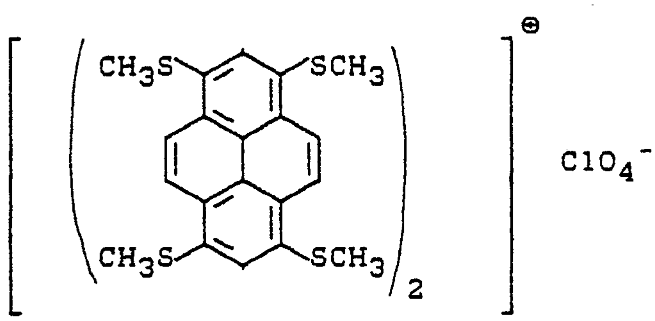

in Form eines schwarzen Kristallpulvers erhalten. Die Leitfähigkeit des CT-Komplexes beträgt 5,1 x $10^{-2}$ S/cm; FP.: 230° C (unter Verpuffung).

Beispiel 25

Die Suspension von 772 mg (2 mMol) 1,3,6,8-Tetramethylmercaptopyren in 1 ml Tetrahydrofuran wird mit 1 ml Wasser, 0,3 ml Schwefelsäure (98 %ig) und 1 g Tetrabutylammoniumperchlorat (3 mMol) versetzt. Das so erhaltene Gemisch wird mit einer Lösung von 32 mg (0,2 mMol) Kaliumpermanganat in 5 ml Wasser versetzt. Es bildet sich sofort ein schwarzer Niedrschlag. Dieser wird abgesaugt, mit Wasser gewaschen

und getrocknet.

Es werden 0,77 g (= 88 % der Theorie) des CT-Komplexes der in Beispiel 24 angegebenen Formel erhalten. Die Leitfähigkeit des Pulvers beträgt 0,35 S/cm; FP.: 230° C (unter Verpuffung).

Beispiel 26

Es wird wie in Beispiel 24 verfahren, nur werden anstelle des $H_2O_2$ 114 mg (0,5 mMol) Ammoniumperoxodisulfat verwendet. Es werden 0,205 g (=95 % der Theorie) des CT-Komplexes der Formel

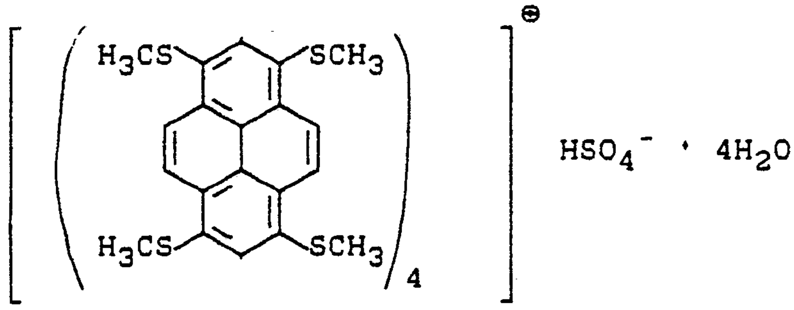

in Form eines schwarzen Kristallpulvers erhalten. Die Leitfähigkeit des CT-Komplexes beträgt $1 \times 10^{-2}$ S/cm.

Beispiel 27

Die Suspension von 772 mg (5 mMol) 1,3,6,8-Tetramethylmercaptopyren in 1 ml Tetrahydrofuran wird mit 3 ml Wasser, 0,3 ml Schwefelsäure (98 %ig) und 1 g (6 mMol) p-Toluolsulfonsäure versetzt. Dieses Gemisch wird anschließend tropfenweise mit einer Lösung von 32 mg (0,2 mMol) Kaliumpermanganat in 5 ml Wasser versetzt. Der entstehende Niederschlag wird abgesaugt, mit Wasser gewaschen und getrocknet.

Es werden 0,45 g (= 47 % der Theorie) des CT-Komplexes der Formel

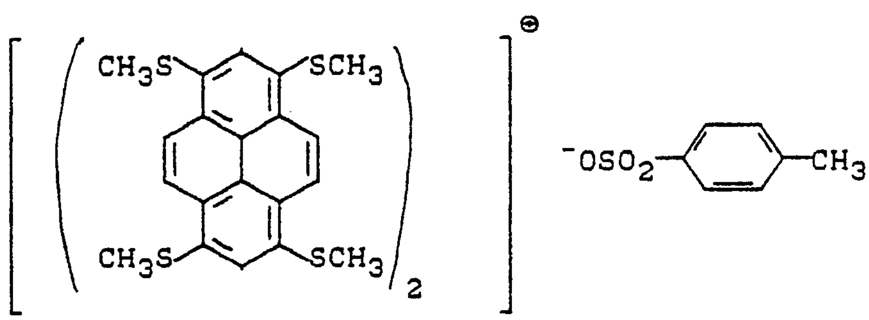

in Form eines schwarzen Kristallpulvers erhalten. Die elektrische Leitfähigkeit des CT-Komplexes beträgt 1,25 S/cm; FP.: 270° C (unter Zersetzung).

**Ansprüche**

1. Mercaptopyrene der Formel

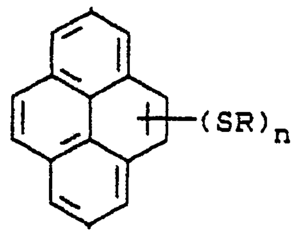

(I) ,

in der
n eine ganze Zahl von 3 bis 10, bedeutet und
R für einen gegebenenfalls substituierten Alkyl-, Alkenyl-, Alkinyl, Cycloalkyl-, Aralkyl-oder Aryl-Rest steht.

2. Mercaptopyrene gemäß Anspruch 1, daß dadurch gekennzeichnet ist, daß
n 3 oder 4 bedeutet und
R für einen gegebenenfalls durch $C_1$-$C_4$-Alkyl, $C_1$-$C_4$-Alkoxy, und/oder Halogen substituierten $C_1$-$C_{22}$-Alkyl-, Benzyl- oder Phenyl-Rest steht.

3. Verfahren zur Herstellung von Mercaptopyrenen der Formel

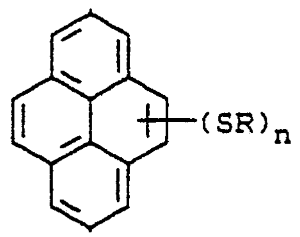

(I) ,

in der
n eine ganze Zahl von 3 bis 10, bedeutet und
R für einen gegebenenfalls substituierten Alkyl-, Alkenyl-, Alkinyl-, Cycloalkyl-, Aralkyl-oder Aryl-Rest steht, dadurch gekennzeichnet, daß man Halogenpyrene der Formel

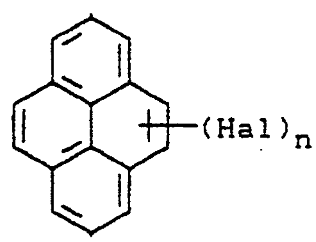

(II) ,

in der
n die unter Formel (I) angegebene Bedeutung hat und
Hal für Halogen steht,
mit Mercaptiden der Formel
$K^{m\oplus}$ $(SR)_m$ (III) ,
in der
R die unter Formel (I) angegebene Bedeutung hat und
$K^{m\oplus}$ für ein m-wertiges Kation steht,
in einem stark polaren, aprotischen organischen Lösungsmittel bei Temperaturen von 0 bis 150° C umsetzt.

4. Verwendung der Mercaptopyrene gemäß Anspruch 1 oder 2 zur Herstellung von CT-Komplexen der Formel

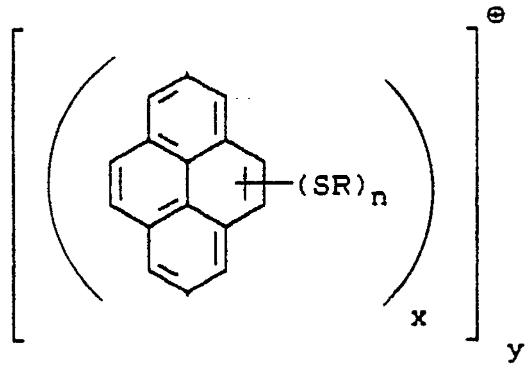

$A^{y\ominus}$ (IV) ,

in der
n eine ganze Zahl von 3 bis 10 bedeutet,
R für einen gegebenenfalls substituierten Alkyl-, Alkenyl-, Alkinyl-, Cycloalkyl-, Aralkyl- oder Aryl-rest steht,
x die Gesamtzahl der in einem 1-wertigen Mercaptopyren-Kation enthaltenen neutralen und geladenen Mercaptopyren-Einheiten angibt und eine ganze oder gebrochene Zahl von 1 bis 10 ist,
$A^{\ominus}$ für ein Anion steht und
y die Zahl der negativen Ladungen des Anions und die zur Neutralisation dieser negativen Ladungen erforderliche Zahl 1-wertiger Mercaptopyren-Kationen bedeutet und eine ganze Zahl von 1 bis 3 ist.

5. CT-Komplexe der Formel

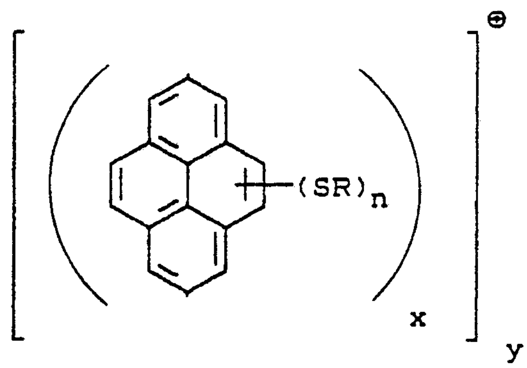

$A^{y\ominus}$ (IV) ,

in der
n eine ganze Zahl von3 bis 10 bedeutet,
R für einen gegebenenfalls substituierten Alkyl-, Alkenyl-, Alkinyl-, Cycloalkyl-, Aralkyl- oder Aryl-Rest steht,
x die Gesamtzahl der in einem 1-wertigen Mercaptopyren-Kation enthaltenen neutralen und geladenen Mercaptopyren-Eiheiten angibt und eine ganze oder gebrochene Zahl von 1 bis 10 ist,
$A^{\ominus}$ für ein Anion steht und
y die Zahl der negativen Ladungen des Anions und die zur Neutralisation dieser negativen Ladungen erforderliche Zahl 1-wertiger Mercaptopyren-Kationen bedeutet und eine ganze Zahl von 1 bis 3 ist.

6. CT-Komplexe gemäß Anspruch 5, dadurch gekennzeichnet, daß
n die Zahl 3 oder 4 bedeutet,
R für einen gegebenenfalls durch $C_1$-$C_4$-Alkyl, $C_1$-$C_4$-Alkoxy und/oder Halogen substituierten $C_1$-$C_{22}$-Alkyl-Benzyl- oder Phenyl-Rest steht,
x eine ganze oder gebrochene Zahl von 1 bis 5 ist und
y 1 ist.